(19) Europäisches Patentamt
European Patent Office
Office européen des brevets

(11) **EP 3 158 921 A1**

(12) **EUROPEAN PATENT APPLICATION**

(43) Date of publication:
**26.04.2017 Bulletin 2017/17**

(51) Int Cl.:
**A61B 3/113** (2006.01)     **A61B 5/18** (2006.01)

(21) Application number: **16194340.2**

(22) Date of filing: **18.10.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **23.10.2015 JP 2015209261**

(71) Applicant: **FUJITSU LIMITED**
**Kawasaki-shi Kanagawa 211-8588 (JP)**

(72) Inventors:
• **Yoshioka, Takahiro,**
  **Kanagawa, 211-8588 (JP)**
• **Nakashima, Satoshi**
  **Kanagawa, 211-8588 (JP)**

(74) Representative: **Hoffmann Eitle**
**Patent- und Rechtsanwälte PartmbB**
**Arabellastraße 30**
**81925 München (DE)**

(54) **LINE OF SIGHT DETECTION SYSTEM AND METHOD**

(57)     A line of sight detection system includes a light source that emits light toward a subject, a light detector configured to detect reflected light from the subject, a control unit configured to change an emission pattern of the light emitted from the light source toward the subject, an identification unit configured to identify a line of sight position of the subject based on a change of the reflected light from the subject when the emission pattern of the light is changed, and an output unit configured to output information of the line of sight position.

FIG. 3

**Description**

FIELD

The embodiments discussed herein are related to a technique for detecting a line of sight of a subject.

BACKGROUND

There is a technique for detecting a line of sight of a person, who is a subject, by a corneal reflection method using a near infrared light source and a near infrared camera (for example, Takehiko Ohno et al., An Eye Tracking System Based on Eye Ball Model-Toward Realization of Gaze Controlled Input Device-, Information Processing Society of Japan Technical Reports 2001-HI-93, 2001, pp47-54). In the corneal reflection method, a reflection is generated on a cornea using a near infrared light source, and a center position of the corneal reflection and a center position of a pupil are obtained from image processing. In the corneal reflection method, the line of sight of a subject is detected from a relationship between the center position of the corneal reflection and the center position of a pupil.

Also, if a subject whose line of sight is to be detected is a person who is wearing glasses, for example, near infrared light irradiated from a near infrared light source is reflected on the lens surface of the glasses. Following, a reflection on the lens surface of glasses is referred to as a glasses reflection. Various proposals have been made on the line of sight detection techniques in consideration of a glasses reflection (for example, Japanese Laid-open Patent Publication Nos. 2006-167256 and 2003-339642).

SUMMARY

TECHNICAL PROBLEM

In an image captured in a state in which a glasses reflection occurs, the glasses reflection overlaps a corneal reflection and a pupil, so that it sometimes becomes difficult to detect the corneal reflection and the pupil from the image. Thus, with a technique disclosed in the present embodiment, it is desirable to detect a line of sight of a subject who is wearing glasses.

SOLUTION TO PROBLEM

According to an aspect of the invention, a line of sight detection system includes a light source that emits light toward a subject, a light detector configured to detect reflected light from the subject, a control unit configured to change an emission pattern of the light emitted from the light source toward the subject, an identification unit configured to identify a line of sight position of the subject based on a change of the reflected light from the subject when the emission pattern of the light is changed, and an output unit configured to output information of the line of sight position.

BRIEF DESCRIPTION OF DRAWINGS

FIG. 1 is a diagram illustrating a configuration of a line of sight detection system according to the present embodiment;
FIG. 2A and FIG. 2B are diagrams for explaining a relationship between an irradiation area irradiated from a light source, and a corneal reflection and a glasses reflection;
FIG. 3 is a functional block diagram illustrating functions of an identification unit;
FIG. 4 is an example of a configuration of an association table;
FIG. 5 is a flowchart of learning processing;
FIG. 6 is a flowchart (1 of 2) of line of sight detection processing;
FIG. 7 is a flowchart (2 of 2) of line of sight detection processing;
FIG. 8A and FIG. 8B are diagrams for explaining the advantages of line of sight detection according to the present embodiment; and
FIG. 9 is a hardware configuration of a line of sight detection system including a line of sight detection device.

DESCRIPTION OF EMBODIMENTS

Following, a detailed description will be given of embodiments of the line of sight detection technique according to the present disclosure with reference to the drawings. In this regard, this disclosure is not limited to the embodiments.

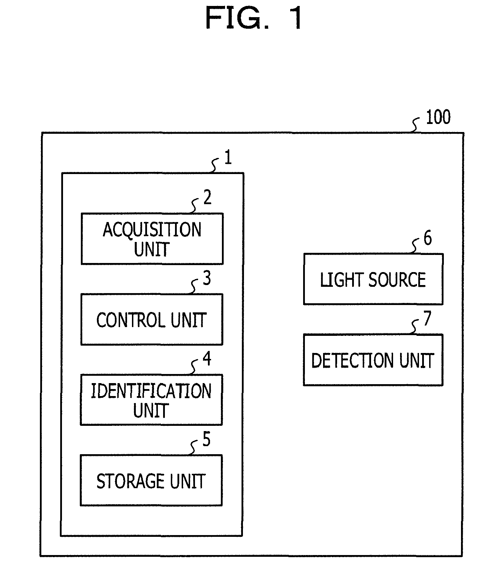

FIG. 1 is a diagram illustrating a configuration of a line of sight detection system according to the present embodiment. As illustrated in FIG. 1, a line of sight detection system 100 includes a line of sight detection device 1, a light source 6, and a detection unit 7. The light source 6 irradiates light of a predetermined wavelength toward a subject. The detection unit 7 detects light reflected from the subject, which is irradiated from the light source 6.

The detection unit 7 is a camera having a sensitivity to light of the predetermined wavelength, which is irradiated by the light source 6, for example. Accordingly, the detection unit 7 captures images of the subject so as to detect reflected light from the subject, which is irradiated from the light source 6. In this regard, the subject is a person whose line of sight is to be detected. Also, the subject represents the subject and another objects (including glasses).

In the present embodiment, near infrared light that is invisible to the subject is used as light of the predetermined wavelength. Accordingly, the camera as an example of the detection unit 7 is a near infrared light camera, the light source 6 is a light emitting diode (LED) that emits near infrared light. The images obtained by the detection unit 7 as a detection result are near infrared images. The near infrared image includes the state of the subject in brightness in accordance with the intensity of the reflection of near infrared light irradiated from the light source 6 and near infrared light (for example, natural light or fluorescent light) irradiated from another light source.

The line of sight detection device 1 detects a line of sight of the subject. In this regard, the line of sight detection device 1 is a computer including a processor that executes various kinds of processing and a memory that stores information, for example.

In the line of sight detection system 100, the light source 6 and the detection unit 7 are coupled to the line of sight detection device 1. However, the coupling mode thereof may be a mode of wireless communication in addition to a wired coupling. For example, when line of sight detection processing is started, under the control of the line of sight detection device 1, the detection unit 7 captures images of the subject at certain time intervals and outputs the captured images to the line of sight detection device 1. Also, when line of sight detection processing is started, the line of sight detection device 1 performs control so as to turn on the light source 6.

In the present embodiment, the line of sight detection device 1 performs line of sight detection processing on the near infrared image obtained from the detection unit 7 so as to detect a line of sight of the subject. For example, the line of sight detection device 1 detects a center position of the corneal reflection of the light irradiated from the light source 6 and a center position of the pupil of the subject using the near infrared image so as to detect a line of sight.

The processing result by the line of sight detection device 1 is utilized for a marketing analysis, for example. Specifically, if the detection unit 7 and the light source 6 are disposed on a shelf or a plate of goods in a store, the line of sight detection device 1 detects lines of sight of customers (subjects) who have visited the store. In the marketing analysis, an estimation is made as to which product has interested the customers from the line of sight detection result. For example, it is possible to grasp the products that have interested a large number of customers from the output (line of sight detection result) of the line of sight detection device 1.

Also, the processing result by the line of sight detection device 1 is utilized for dangerous driving detection, for example. Specifically, if the detection unit 7 is disposed at the place capable of capturing images of a driver who sits on a driver's seat, and the light source 6 is disposed at the place capable of irradiating light onto the driver, the line of sight detection device 1 detects the line of sight of the driver (subject). For the purpose of dangerous driving detection, estimations are made as to whether or not the driver pays attention to various directions, and whether or not there is a risk of drowsy driving from the line of sight detection result.

Further, as illustrated in FIG. 1, the line of sight detection device 1 includes an acquisition unit 2, a control unit 3, an identification unit 4, and a storage unit 5. The acquisition unit 2 obtains the detection result by the detection unit 7 in sequence, and inputs the detection result into the identification unit 4. For example, the acquisition unit 2 obtains, from the detection unit 7 in sequence, the image information of the near infrared image in which the face of the subject is captured. The image information includes luminance information indicating the intensity of the reflection of near infrared light in each pixel. In this regard, if the detection unit 7 and the line of sight detection device 1 perform wireless communication, the acquisition unit 2 functions as a communication unit.

The control unit 3 changes the irradiation area of light irradiated from the light source 6 on the subject. For example, if the light source 6 is a light source including a plurality of light emitting elements, the control unit 3 controls turning on or turning off of each of the light emitting elements in order to control the number of the elements to be turned on. By controlling the number of light emitting elements to be turned on, the control unit 3 may control to change the irradiation area of light irradiated from the light source 6 on the subject. Also, if the line of sight detection system 100 is provided with an aperture disposed between the light source 6 and the subject, the control unit 3 may control the size of the aperture so as to change the irradiation area of light irradiated from the light source 6 on the subject.

The identification unit 4 identifies the line of sight position of the subject based on a change of light reflected from the subject when the irradiation area of light is changed. That is to say, the identification unit 4 detects the line of sight of the subject. For example, the identification unit 4 detects a corneal reflection and a pupil of the subject using a first image captured when the irradiation area is in a first state and a second image captured when the irradiation area is in a second state so as to identify the line of sight position of the subject.

As described later, it is assumed that the irradiation area in the first state is larger than the irradiation area in the second state. For example, if the light source 6 is a light source including an array of a plurality of light emitting elements, the first state is a state in which all the light emitting elements are turned on. Also, the second state is a state in which some of the light emitting elements are turned on (the other light emitting elements are turned off). In this regard, some of the light emitting elements to be turned on in the second state are light emitting elements that do not have a discrete positional relationship with each other, but that exist in a gathered manner, for example adjacently, or the like. Also, for example, if the line of sight detection system 100 is provided with an aperture, the first state is a state of the widest aperture. The second state is a state of a narrow aperture.

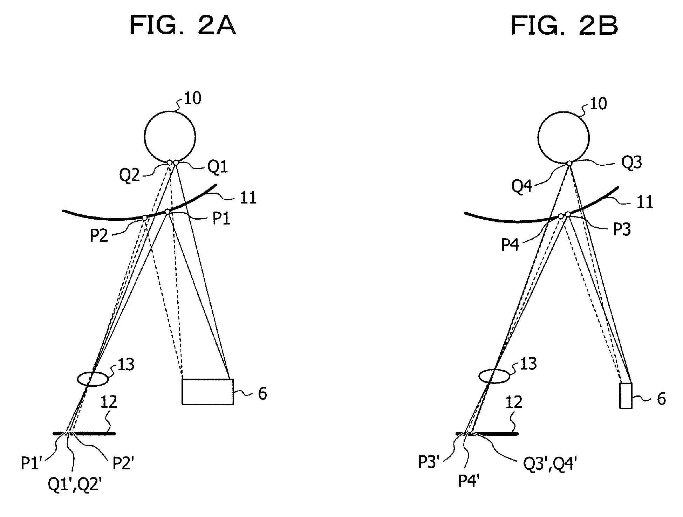

Here, in the present embodiment, when an irradiation area of light irradiated from the light source 6 onto the subject is changed, the fact that a change in the size of the glasses reflection is larger than a change in the size of the corneal reflection is used. FIG. 2A and FIG. 2B are diagrams for explaining a relationship between an irradiation area irradiated from a light source, and a corneal reflection and a glasses reflection.

FIG. 2A and FIG. 2B schematically illustrate an eyeball 10 of a subject, a lens surface 11 of glasses, and a light source 6. Note that the irradiation area irradiated from the light source 6 in FIG. 2B is smaller than the irradiation area irradiated from the light source 6 in FIG. 2A. Also, the plane 12 illustrated in FIG. 2A and FIG. 2B schematically illustrates a projection plane of the detection unit 7 (camera). Further, the plane 13 illustrated in FIG. 2A and FIG. 2B schematically illustrates a lens of the detection unit 7 (camera).

Here, light irradiated from the light source 6 is reflected at various places on the lens surface 11 of the glasses. However, the reflected light detected by the detection unit 7 (camera) is the light reflected at an intersection point between a line connecting the middle point between the center position of the camera lens and the position of the light source 6 and the center of the spherical surface of the lens surface 11 of the glasses, and the lens surface 11 of the glasses. That is to say, when the light source 6 has the irradiation area illustrated in FIG. 2A, light reflected at P1 on the lens surface 11 of the glasses is detected by the detection unit 7 out of the light irradiated from the rightmost end of the light source 6. Also, light reflected at P2 on the lens surface 11 of the glasses is detected by the detection unit 7 out of the light irradiated from the leftmost end of the light source 6. From the above, in FIG. 2A, a glasses reflection occurs from P1 to P2 on the lens surface 11 of the glasses.

In the same manner, light irradiated from the light source 6 is reflected at various places on the eyeball 10. However, the reflected light detected by the detection unit 7 (camera) is the light reflected at an intersection point between a line connecting the middle point between the center position of the camera lens and the position of the light source 6 and the center of the sphere of the eyeball 10, and the eyeball 10. That is to say, when the light source 6 has the irradiation area illustrated in FIG. 2A, light reflected at Q1 on the eyeball 10 is detected by the detection unit 7 out of the light irradiated from the rightmost end of the light source 6. Also, light reflected at Q2 on the eyeball is detected by the detection unit 7 out of the light irradiated from the leftmost end of the light source 6. From the above, in FIG. 2A, a corneal reflection occurs from Q1 to Q2 on the eyeball 10 of the subject.

Further, in the projection plane 12 in FIG. 2A, the corneal reflection (from Q1' to Q2') is included in the glasses reflection (from P1' to P2'). That is to say, in the image captured in the state of FIG. 2A, the target corneal reflection is hidden by the glasses reflection, and thus it is not possible to detect the corneal reflection.

On the other hand, in FIG. 2B, a glasses reflection occurs from P3 to P4 on the lens surface 11 of the glasses. Also, a corneal reflection occurs from Q3 to Q4 on the eyeball 10 of the subject. Further, in the projection plane 12 in FIG. 2B, the glasses reflection (from P3' to P4') and the corneal reflection (from Q3' to Q4') are separated. In the image captured in the state in FIG. 2B, the glasses reflection that has become small and the corneal reflection are captured in a state of not overlapping with each other. That is to say, it is possible to detect the target corneal reflection.

As described above, depending on the difference in the curvature between the glasses and the eyeball, and the difference in the radius between the glasses and the eyeball, the glasses reflection becomes significantly smaller in the state in FIG. 2B, in which the irradiation area is small, than in the state in FIG. 2A, in which the irradiation area is large. On the other hand, although the corneal reflection also becomes smaller in the state in FIG. 2B than in the state in FIG. 2A, the corneal reflection is less influenced by a change of the irradiation area than the glasses reflection.

Referring back to FIG. 1, the storage unit 5 stores various kinds of information that is demanded for the line of sight detection processing according to the present embodiment. For example, the storage unit 5 stores the detection result (image information) by the detection unit 7, which is obtained by the acquisition unit 2, and an association table described later, or the like.

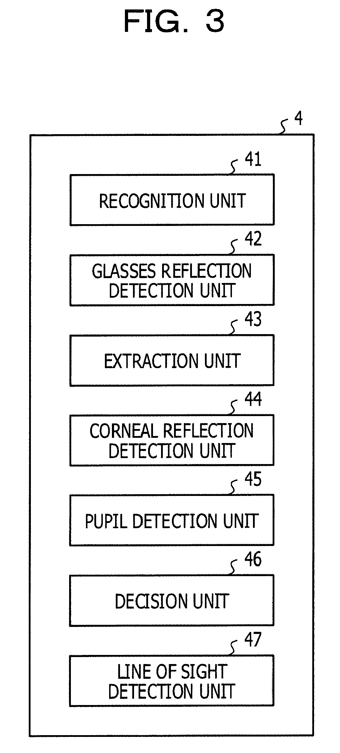

Next, a detailed description will be given of the identification unit 4. FIG. 3 is a functional block diagram illustrating functions of the identification unit. The identification unit 4 includes a recognition unit 41, a glasses reflection detection unit 42, an extraction unit 43, a corneal reflection detection unit 44, a pupil detection unit 45, a decision unit 46, and a line of sight detection unit 47.

The recognition unit 41 recognizes a subject and a specific part of the subject from images. For example, the recognition unit 41 recognizes a face of the subject from each of the first image captured when the irradiation area is in the first state and the second image captured when the irradiation area is in the second state. In this regard, the conventional face recognition technique is used for the face recognition.

Further, the recognition unit 41 may recognize a specific part from a region (face region) recognized as a face. In a specific part recognition, the conventional recognition technique is used for recognizing parts of a human face. In the present embodiment, the recognition unit 41 searches a face region for a region matching the features of an eye that are learned in advance so as to recognize an eye. The recognition unit 41 then generates eye region information indicating an eye region in the image and stores the eye region information in the storage unit 5. In this regard, the eye region information is information including, for example information indicating a range of an eye region and information indicating a center position (or a position of the center of gravity) of the eye region.

If it is not possible to detect a corneal reflection from the first image that was captured in a state, in which the irradiation area irradiated from the light source 6 is wider, by the corneal reflection detection unit 44 described later, the glasses reflection detection unit 42 detects a glasses reflection region from the first image. For example, the glasses reflection detection unit 42 identifies a pixel region having a luminance value of a predetermined value (for example, 240) or more from the eye region in the first image based on the eye region information input from the recognition unit 41, and if the region includes pixels of a predetermined number (for example, 30 pixels) or more, the glasses reflection detection unit 42 detects the region as a glasses reflection region.

The glasses reflection detection unit 42 stores the information on the detected glasses reflection region into the storage unit 5. In this regard, the information on the glasses reflection region includes, for example information indicating the range of the glasses reflection region and information indicating the center position (or a position of the center of gravity) of the glasses reflection region.

Next, the extraction unit 43 extracts a feature point from each of the first image and the second image. For example, the extraction unit 43 extracts a corner as a feature point from the eye region in the first image using the Harris operator or the Fast operator. The extraction unit 43 then stores the position of the extracted feature point into the storage unit 5. The extraction unit 43 performs the same operations for the second image. In this regard, the extraction unit 43 extracts, as feature points, a corner of the frame of glasses, an inner corner of the eye, and the like, for example.

The corneal reflection detection unit 44 detects a corneal reflection from the first image captured in the state in which the irradiation area irradiated from the light source 6 is wider. However, if it is not possible to detect a corneal reflection from the first image, the corneal reflection detection unit 44 detects a corneal reflection from the second image captured in the state of a narrower irradiation area.

When the corneal reflection detection unit 44 detects a corneal reflection from the second image, the corneal reflection detection unit 44 uses the information of the feature point extracted by the extraction unit 43. Specifically, the corneal reflection detection unit 44 associates the feature point extracted from the first image with the feature point extracted from the second image. The corneal reflection detection unit 44 then calculates a motion vector of the associated feature points. The amount of movement and the movement direction are reflected on the motion vector when the subject moves from a point in time of capturing the first image to a point in time of capturing the second image.

For example, if a feature point $(x, y)$ in the first image is associated with a feature point $(x', y')$ in the second image, its motion vector V is expressed by the expression 1 described below.

$$V = (x' - x, \ y' - y) \ \ldots \ \text{expression 1}$$

The corneal reflection detection unit 44 then estimates the center position of the glasses reflection in the second image in accordance with the center position of the glasses reflection region detected from the first image and the motion vector. That is to say, the corneal reflection detection unit 44 estimates the position where the glasses reflection may occur in consideration of the movement of the subject.

Specifically, the corneal reflection detection unit 44 reflects the motion vector V on the position $(Px, Py)$ of the glasses region in the first image and estimates the position $(Px', Py')$ of the glasses region in the second image.

$$Px' = Px + (x' - x), \ Py' = Py + (y' - y) \ \ldots \ \text{expression 2}$$

The corneal reflection detection unit 44 then detects a set of high luminance pixels that exist at the place other than the surroundings of the estimated position of the glasses reflection from the second image and detects the set as a corneal reflection. In this regard, the surroundings of the estimated position of the glasses reflection refer to within a radius of 10 pixels, for example.

In the present embodiment, at the time of capturing the second image, the irradiation area of the light irradiated from the light source 6 to the subject is reduced so that the area in which a glasses reflection is generated is reduced in order to separate from a corneal reflection. The corneal reflection is a set of high luminance pixels similar to the glasses reflection, and thus the corneal reflection and the glasses reflection have to be distinguished.

However, it is lees effective if the same detection method of a glasses reflection region is applied to the second image as the detection method that is applied by the glasses reflection detection unit 42 to the first image. Because by reducing the irradiation area, the glasses reflection in the second image itself becomes small. Accordingly, there is a high possibility that it is not possible to detect a glasses reflection from the second image using the algorithm (for example, detecting a region of a set of 30 or more high luminance pixels) executed by the glasses reflection detection unit 42.

Thus, after the irradiation area of the light irradiated from the light source 6 is reduced, the corneal reflection detection unit 44 estimates the position of the glasses reflection in the second image in consideration of the motion vector and the glasses reflection position in the first image. The corneal reflection detection unit 44 then detects a set of high luminance pixels that exist at the place other than the surroundings of the estimated position of the glasses reflection from the second image as a corneal reflection.

In this regard, the corneal reflection detection unit 44 may estimate not only the position (the center position and the position of the center of gravity) of the glasses reflection region, but the glasses reflection region in accordance with the irradiation area. For example, if the irradiation area is made one quarter from the first state to the second state, it is estimated that a glasses reflection that is one quarter the glasses reflection region in the first image occurs at the estimated position of the glasses reflection region in the second image. The corneal reflection detection unit 44 then detects a set of high luminance pixels that exist in a region other than the estimated glasses reflection region as a corneal reflection.

Next, when a corneal reflection is detected from the first image, the pupil detection unit 45 detects a pupil from the first image. Also, when a corneal reflection is not detected from the first image and when a corneal reflection is detected from the second image, the detection unit 45 detects a pupil from the second image. In this regard, the conventional pupil detection technique is applied to the detection of a pupil. For example, the pupil detection unit 45 performs matching with a template corresponding to the shape of a pupil.

Next, if the corneal reflection could not be detected from the first image, the decision unit 46 decides the irradiation area to be irradiated from the light source 6 before the detection unit 7 captures the second image, and inputs the decided information to the control unit 3. The control unit 3 controls the irradiation area based on the control information input from the decision unit 46. For example, if the light source 6 includes a plurality of light emitting elements, the control information is the number of light emitting elements to be turned on and their positions. If the line of sight detection system 100 has an aperture, the control information is the size of the aperture.

Here, a description will be given of a method of deciding the irradiation area by the decision unit 46. First, the decision unit 46 obtains the distance between the subject or a part (eye) of the subject and the detection unit 7. The distance may be measured by a distance sensor, or may be estimated by the following method from the first image.

For example, the decision unit 46 obtains the distance (pixels) between the pupil of the right eye and the pupil of the left eye in the first image. For example, it is assumed to be understood that in the specification of a certain detection unit 7, when the distance between the subject and the detection unit 7 (camera) is 60 cm, the distance between both pupils is 30 pixels. At this time, the distance between the subject and the detection unit 7 is estimated from this association relationship and the obtained distance of both pupils from the first image. In this regard, the shorter the distance between the subject and the detection unit 7, the longer the distance between both pupils. Accordingly, the relationship between the two is inversely proportional. Also, if the distance between the subject and the detection unit 7 is obtained from the first image, the distance between the pupils may not be used, and the distance between inner corners of both eyes, or the like may be used.

The decision unit 46 then estimates the size of the corneal reflection to be originally detected in the first image in which a corneal reflection was unable to be detected from the distance between the subject and the detection unit 7. At this time, the decision unit 46 uses the association relationship between the distance of the subject and the detection unit 7, and the size of the corneal reflection. The association relationship is learned in advance, and an association table in which the association relationship is described is stored in the storage unit 5.

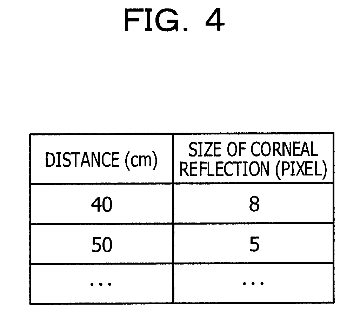

FIG. 4 is an example of the configuration of the association table. The association table stores the distance between the subject and the detection unit 7, and the size of the corneal reflection in the image captured at the distance in association with each other. At learning time, light of the light source 6 is irradiated onto a subject located at a predetermined distance from the detection unit 7, and the image captured by the detection unit 7 is used. At this time, the detection unit 7 captures a plurality of images while the subject is changing the distance with the detection unit 7. The distance when each image is captured and the size of the corneal reflection detected from each image is learned as the association relationship. In this regard, at learning time, it is preferable that the irradiation area of light from the light source 6 onto the subject match the irradiation area in the first state.

The decision unit 46 refers to the association table so as to estimate, for example that the size of the corneal reflection to be originally detected is "5 (pixel)" if the distance between the subject and the detection unit 7 is "50 (cm)".

Next, the decision unit 46 decides how much the irradiation area is to be reduced in order for the glasses reflection in the first image in which a glasses reflection occurs to be reduced to the size corresponding to the corneal reflection to be originally detected. That is to say, the decision unit 46 decides to what fraction of the irradiation area (the first state) at the time of capturing the first image the irradiation area is to be reduced in accordance with the ratio between the size of the glasses reflection in the first image and the estimated corneal reflection. Here, the decided irradiation area becomes the second state.

For example, if the size of the glasses reflection in the first image is 40 pixels, and the size of the estimated corneal reflection is 5 pixels, the decision unit 46 decides the irradiation area at the time of capturing the second image to be one eighth the irradiation area at the time of capturing the first image. In this case, for example if the light source 6 includes a plurality of light emitting elements, the decision unit 46 decides the number of light emitting elements to be turned on to be one eighth. In this regard, it is assumed that the storage unit 5 stores the number of light emitting elements included in the light source 6 in advance. Also, if the number of light emitting elements to be turned on, which is decided from the ratio between the size of the glasses reflection in the first image and the estimated corneal reflection, is not an integer, the decision unit 46 ought to decide the number of light emitting elements to be turned on to be an integer by rounding down decimal places, or the like, for example.

Next, the line of sight detection unit 47 detects the line of sight position of the subject using the center position of the corneal reflection and the center position of the pupil, which was detected from the first image or the second image. In this regard, the conventional corneal reflection method is applied to the method of deciding the line of sight position from the center position of the corneal reflection and the center position of the pupil.

Next, a description will be given of the flow of learning processing for creating an association table according to the present disclosure. In this regard, the learning processing may be executed by the line of sight detection system 100 or may be executed by another computer.

In the former case, the line of sight detection system 100 further includes a learning unit (not illustrated in the FIG. 1), and the learning unit executes the learning processing in cooperation with another processing units. The learning unit then stores the learning result in the association table (the storage unit 5). In this regard, the learning unit is a processing unit that executes the learning processing and has functions corresponding to the acquisition unit 2, the control unit 3 and the identification unit 4 in the line of sight detection system 100.

In the latter case, another computer executes the learning processing and outputs the association relationship corresponding to the learning result to the line of sight detection system 100. The line of sight detection system 100 stores the association relationship, which is input, into the association table (the storage unit 5).

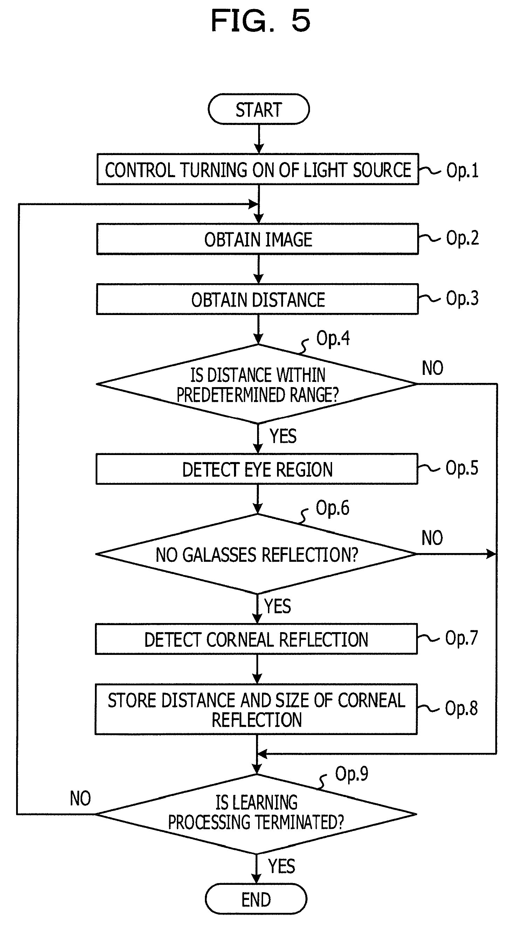

FIG. 5 is a flowchart of the learning processing. Here, a description will be given of an example in which the line of sight detection system 100 executes the learning processing. First, the control unit 3 controls the light source 6 in order to turn on the light source 6 (Op. 1). Preferably, the control unit 3 turns on the light source 6 so as to become the first state in which the irradiation area irradiated by the light source 6 is sufficiently large. For example, if the light source 6 includes a plurality of light emitting elements, the learning unit turns on all the light emitting elements.

Next, the learning unit obtains an image from the detection unit 7 (Op. 2). The learning unit then obtains the distance between the subject and the detection unit 7 when the image is captured (Op. 3). In this regard, the method of obtaining the distance may be the same as the method of obtaining the distance by the corneal reflection detection unit 44, or the subject or the administrator may input the distance.

The learning unit then determines whether or not the distance between the subject and the detection unit 7 is within a predetermined range. (Op. 4) In this regard, the predetermined range is, for example 40 cm to 70 cm, and a distance suitable for the line of sight detection processing is set. If the distance between the subject and the detection unit 7 is within the predetermined range (Op. 4 YES), the learning unit detects a face region and an eye region from the image (Op. 5). Further, the learning unit determines whether or not there is no glasses reflection in the eye region (Op. 6). If there is no glasses reflection (Op. 6 YES), the learning unit detects a corneal reflection (Op. 7). Further, the learning unit stores the distance obtained in Op. 3 and the size of the corneal reflection detected in Op. 7 in association with each other in the storage unit 5 (Op. 8).

On the other hand, if the distance between the subject and the detection unit 7 is not within the predetermined range (Op. 4 NO) or if there is a glasses reflection (Op. 6 NO) or when the processing in Op.8 is completed, the learning unit determines whether or not to terminate the learning processing (Op. 9). For example, if the size of a corneal reflection in each distance has been learned, or there is an input of terminating the learning processing, the learning unit terminates the learning processing (Op. 9 YES). On the other hand, if the learning processing is not terminated (Op. 9 NO), the learning unit obtains a new image (Op. 2). By the above learning processing, an association relationship (association table) as illustrated in FIG. 4 is generated.

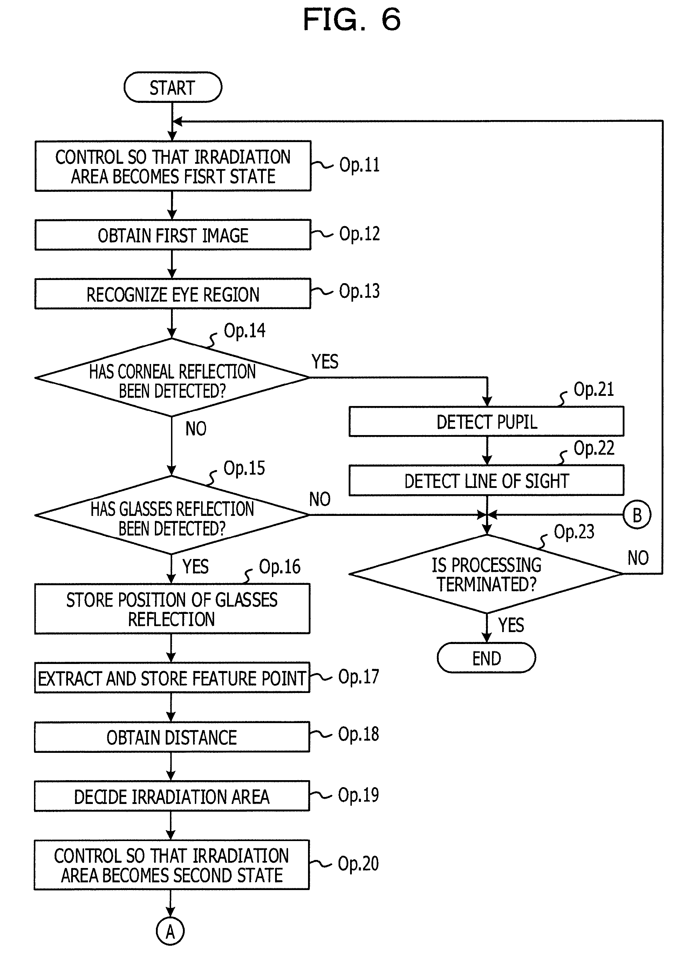

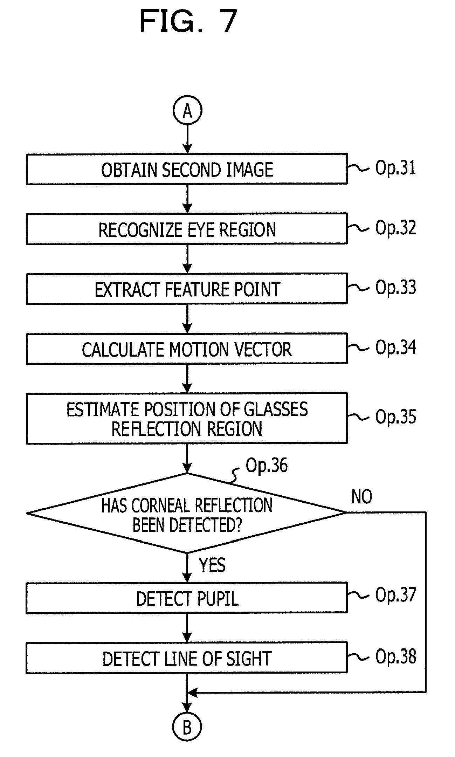

Next, a description will be given of the flow of line of sight detection processing according to the present embodiment. FIG. 6 and FIG. 7 are the flowcharts of the line of sight detection processing.

First, the control unit 3 performs control so that the irradiation area irradiated from the light source 6 becomes the first state (Op. 11). For example, if the light source 6 includes a plurality of light emitting elements, the control unit 3 performs control so as to turn on all the light emitting elements. Also, if the line of sight detection system 100 has an aperture (not illustrated in FIG. 1), the control unit 3 performs control so that the aperture becomes the maximum size.

The acquisition unit 2 obtains the first image captured after the irradiation area of the detection unit 7 irradiated by the light source 6 becomes the first state (Op. 12). In this regard, before Op. 12, the control unit 3 may control the light source 6 and then may further give an instruction to capture the first image to the detection unit 7.

Next, the recognition unit 41 detects a face region from the first image, and further detects an eye region from the face region (Op. 13). For example, the recognition unit 41 executes the face recognition processing on the first image, and if a face is recognized, the eye recognition processing is executed on the face region.

Next, the corneal reflection detection unit 44 determines whether or not a corneal reflection has been detected from the eye region in the first image (Op. 14). For example, the corneal reflection detection unit 44 determines whether or not a region including a set of about 3 to 10 pixels having a luminance value of 240 or more has been detected from the eye region.

If a corneal reflection is detected (Op. 14 YES), the pupil detection unit 45 further detects a pupil from the eye region in the first image (Op. 21). The line of sight detection unit 47 then detects the line of sight direction and the position of the subject at the point in time when the first image is captured based on the center position of the corneal reflection and the center position of the pupil (Op. 22).

The line of sight detection unit 47 then determines whether or not a series of line of sight detection processing is completed (Op.23). If determined that the line of sight detection processing is completed (Op.23 YES), the series of processing is completed. On the other hand, if determined that the line of sight detection processing is to be continued (Op.23 NO), the line of sight detection device 1 repeats the processing from Op.11.

On the other hand, if a corneal reflection has not been detected (Op.14 NO), the glasses reflection detection unit 42 determines whether or not a glasses reflection has been detected in the eye region (Op.15). For example, the glasses reflection detection unit 42 determines whether or not a region including a set of 30 pixels having a luminance value of 240 or more.

If a glasses reflection has not been detected (Op.15 NO), the line of sight detection unit 47 executes the processing in Op.23. That is to say, it was not possible to detect a corneal reflection in the state in which there is no glasses reflection, and thus the line of sight detection system 100 terminates the line of sight detection processing or attempts to capture the first image once again.

On the other hand, if a glasses reflection has been detected (Op.15 YES), the glasses reflection detection unit 42 stores information indicating the position and the range of the glasses reflection region (Op.16). Next, the extraction unit 43 extracts a feature point from the first image as described above and stores the position of the feature point and the feature information related to the feature point in the storage unit 5 (Op.17). In this regard, the extraction of the feature point may be carried out with an eye region as a target, or may be carried out with a face region as a target. Also, the feature information related to the feature point is color information, luminance information, and the image information of the surroundings of the feature point. The feature information related to the feature point is used for associating the feature points between the first image and the second image.

Next, the decision unit 46 obtains the distance between the subject and the detection unit 7 (Op.18). The decision unit 46 then decides the irradiation area irradiated by the light source 6 (Op.19). For example, the decision unit 46 refers to the association table stored in the storage unit 5 and estimates the size of the corneal reflection that might have originally occurred in the first image based on the distance between the subject and the detection unit 7. Further, the decision unit 46 then decides how much the irradiation area irradiated from the light source 6 ought to be reduced from the first state based on the ratio between the size of the glasses reflection detected from the first image and the size of the estimated corneal reflection. In this regard, here the decided irradiation area becomes the second state.

The control unit 3 then performs control so that the irradiation area irradiated from the light source 6 becomes the second state in accordance with the input from the decision unit 46 (Op.20). Next, the acquisition unit 2 obtains the second image captured by the detection unit 7 from the detection unit 7 after the irradiation area irradiated from the light source 6 becomes the second state (Op.31). In this regard, in advance of Op.31, the control unit 3 may control the light source 6 and then may further instructs the detection unit 7 to capture the second image.

Next, the recognition unit 41 detects a face region from the second image and detects an eye region (Op.32). The extraction unit 43 extracts a feature point from the second image (Op.33). The corneal reflection detection unit 44 then calculates a motion vector (Op.34). For example, the corneal reflection detection unit 44 makes an association between the feature points based on the similarity between the feature information of the feature point extracted from the first image and the feature information of the feature point extracted from the second image. The corneal reflection detection unit 44 then obtains a motion vector V by the expression 1 described before using a change of positions of the associated feature points.

Next, the corneal reflection detection unit 44 estimates the position of the glasses reflection region (Op.35). For example, the corneal reflection detection unit 44 reflects the motion vector V on the position (center position) of the glasses reflection detected from the first image as the expression 2 described before so as to estimate the position (center position) of the glasses reflection in the second image.

The corneal reflection detection unit 44 then determines whether or not a corneal reflection has been detected (Op.36). For example, the corneal reflection detection unit 44 determines whether or not a set of high luminance pixels has been detected in a region other than the estimated position of the surroundings of the glasses reflection region. If a corneal reflection has been detected (Op.36 Yes), the pupil detection unit 45 detects a pupil from the second image (Op.37). Further, the line of sight detection unit 47 detects the direction and the position of the line of sight of the subject based on the center position of the corneal reflection and the center position of the pupil that are detected from the second image (Op.38).

The line of sight detection unit 47 then executes the processing of Op.23 and after that. Also, if a corneal reflection has not been detected (Op.36 NO) the line of sight detection unit 47 executes the processing of Op.23 and after that.

If a glasses reflection occurs, the line of sight detection system 100 according to the present embodiment reduces the irradiation area irradiated by the light source 6 by the above processing so as to enable reduction of the glasses reflection region. The glasses reflection then executes the line of sight detection processing on the second image captured in the state of the reduced area. Accordingly, the possibility of separating the corneal reflection and the glasses reflection becomes high, and thus it becomes highly possible to enable detection of a corneal reflection from the second image.

Further, the line of sight detection system 100 according to the present embodiment estimates the position of the glasses reflection in the second image from the position of the glasses reflection detected in the first image and the motion vector indicating the movement of the subject. Accordingly, it is possible for the line of sight detection system 100 to distinguish the target corneal reflection and the glasses reflection in the second image. Accordingly, it is possible for the line of sight detection system 100 to restrain misdetection of the line of sight, which is caused by mistakenly recognizing a glasses reflection as a corneal reflection.

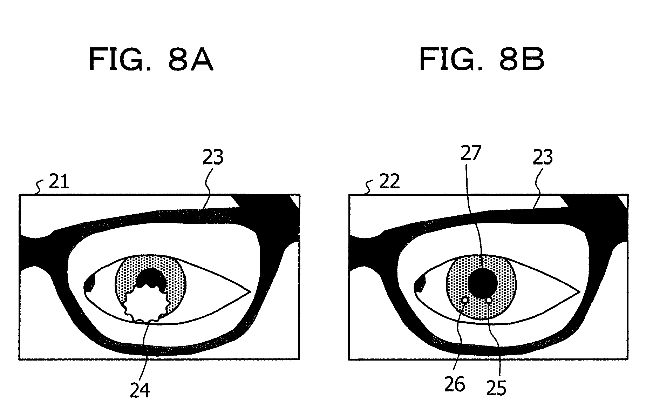

FIG. 8A and FIG. 8B are diagrams for explaining the advantages of line of sight detection according to the present embodiment. FIG. 8A is an enlarged image 21 of an eye region part in the first image. FIG. 8B is an enlarged image 22 of the eye region part in the second image.

In FIG. 8A and FIG. 8B, a subject wears glasses 23. FIG. 8A illustrates the case where the irradiation area irradiated from the light source 6 is in the first state, and thus a glasses reflection 24 is captured in the enlarged image 21. Also, it is in a state in which a corneal reflection is unable to be detected due to the glasses reflection 24. On the other hand, FIG. 8B illustrates the case where the irradiation area irradiated from the light source 6 is in the second state, and thus a glasses reflection 25 that has been reduced is captured in the enlarged image 22. The corneal reflection 26 is then separated from the glasses reflection 25. Further, a pupil 27 is not hidden by the glasses reflection 25, and thus it is possible to recognize the whole.

Also, it is difficult to distinguish the glasses reflection 25 that has been reduced and the corneal reflection 26 in the image. However, with the present embodiment, it is possible to estimate the position of the glasses reflection 25 from the glasses reflection 24 in the first image and the motion vector V, and thus it is possible to estimate the position of the glasses reflection and then to correctly detect the corneal reflection.

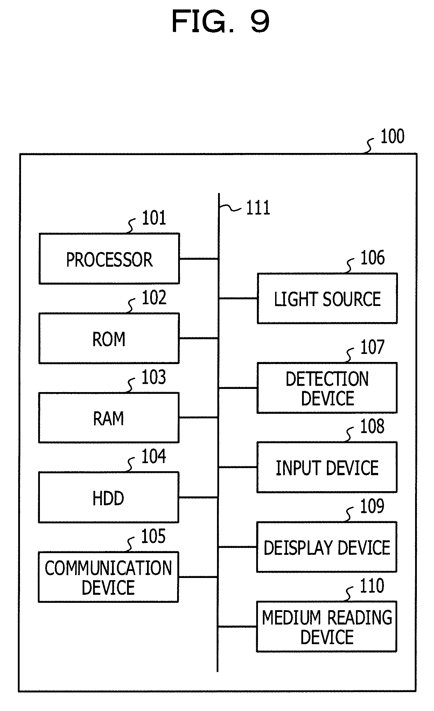

Next, a description will be given of an example of a hardware configuration of the line of sight detection system including the line of sight detection device. FIG. 9 is the hardware configuration of the line of sight detection system including the line of sight detection device. The line of sight detection system 100 performs the line of sight detection processing according to the embodiment.

The line of sight detection device 1 included in the line of sight detection system 100 includes, as a hardware configuration, a processor 101, a read only memory (ROM) 102, a random access memory (RAM) 103, a hard disk drive (HDD) 104, a communication device 105, an input device 108, a display device 109, and a medium reading device 110. Further, the line of sight detection system 100 includes the light source 106 and the detection device 107 in addition to each of the hardware configuration included in the line of sight detection device 1. Each of the units then is mutually coupled via a bus 111. Each of the units is capable of mutually transmitting and receiving data under the control of the processor 101.

A program according to the line of sight detection processing is recorded in a recording medium which is readable by the line of sight detection system 100. The recording medium that is readable by the line of sight detection system 100 is a magnetic recording device, an optical disc, an optical magnetic recording medium, a semiconductor memory, or the like. The magnetic recording device is an HDD, a flexible disk (FD), a magnetic tape (MT), or the like.

The optical disc is a Digital Versatile Disc (DVD), a DVD-RAM, a Compact Disc-Read Only Memory (CD-ROM), a Compact Disc-Recordable/ReWritable (CD-R/RW), or the like. The optical magnetic recording medium is a Magneto-Optical disk (MO), or the like. When a program describing the processing according to each embodiment is distributed, it is thought that for example, a portable recording medium, such as a DVD, a CD-ROM, or the like on which the program is recorded is marketed.

The medium reading device 110 of the line of sight detection system 100, which executes the program according to the present embodiment, reads the program from the recording medium on which the program is recorded. The processor 101 stores the read program into the HDD 104, the ROM 102, or the RAM 103.

The processor 101 performs overall operation control of the line of sight detection device 1. The processor includes an electronic circuit, for example a central processing unit (CPU), or the like.

The processor 101 then reads the program describing the processing according to the present embodiment from the HDD 104 and executes the program so that the processor 101 functions as the control unit 3 and the identification unit 4 in the line of sight detection device 1. The communication device 105 functions as the acquisition unit 2 under the control of the processor 101. The HDD 104 stores various kinds of information and functions as the storage unit 5 under the control of the processor 101. The various kinds of information may be stored in the ROM 102 or the RAM 103, which is capable of being accessed by the processor 101, in the same manner as the program. Further, various kinds of information that is temporarily generated and held in the process of the processing is stored in the RAM 103, for example.

The input device 108 receives the various kinds of information. The input device 108 is, for example a keyboard or a mouse. The display device 109 displays the various kinds of information. The display device 109 is a display, for example.

In this manner, each of the functional units illustrated in FIG. 1 and FIG. 3 are realized by hardware including the processor 101 and the memory (the HDD 102, the ROM 102, or the RAM 103). The processor 101 then reads and executes the program stored in the memory so as to execute the line of sight detection processing illustrated in FIG. 6 and FIG. 7.

Also, the line of sight detection processing illustrated in FIG. 6 and FIG. 7 is sometimes executed in a cloud environment. In this case, the light source 6 and the detection unit 7 are disposed in the space in which a subject exists. The line of sight detection device 1 (one or more servers) that has received a detection result from the detection unit 7 then executes the line of sight detection processing illustrated in FIG. 6 and FIG. 7.

Variations

Here, a description will be given of variations of the present embodiment. In the present embodiment, when a glasses reflection is detected, the decision unit 46 decides the irradiation area irradiated from the light source 6, and the control unit 3 performs control so that the irradiation area of the light source becomes the second state. However, the first state and the second state may be switched over for each detection interval of the detection unit 7 as in the case of the variations. For example, in the variations, the irradiation area irradiated from the light source 6 is enlarged (the first state) and reduced (the second state) for each frame interval of a camera.

First variation

For example, if a glasses reflection has been detected in the first image captured in the first state in the same manner as the embodiment described above, the control unit 3 performs control so that the irradiation area irradiated from the light source 6 becomes the second state. That is to say, if a glasses reflection has been detected in the first image captured in the first state, the irradiation area irradiated from the light source 6 is switched between the first state and the second state between the N-th frame and the (N + 1)-th frame in the same manner as the embodiment described above.

Also, in the first variation, further if a glasses reflection has not been detected in the first image captured in the first state, the control unit 3 performs control so that the irradiation area irradiated from the light source 6 becomes a third state. In this regard, the third state is a specified state, and is a state in which the irradiation area is set to one-half the irradiation area in the first state, for example. The detection unit 7 then captures the reflected light of the light irradiated in the third state (third image). That is to say, the irradiation area irradiated from the light source 6 is switched between the first state and the third state between the N-th frame and the (N + 1)-th frame.

As described above, in the variation, the irradiation area irradiated from the light source in the (N + 1) frame is more reduced than that in the first state regardless of the presence or absence of a glasses reflection. However, if there is a glasses reflection, the irradiation area becomes the second state, whereas if there is no glasses reflection, the irradiation area becomes the third state.

Second variation

Further, the irradiation area irradiated from the light source 6 may be switched between the first state and the second state for each frame. However, unlike the first variation, the "second state" in the second variation is set to a specified value (the third state in the first variation). For example, the irradiation area in the first state is the maximum irradiation area, and the irradiation area in the second state is set to a specified value (for example, one-half the value in the first state).

The line of sight detection system 100 according to this variation switches the irradiation area irradiated by the light source 6 for each frame between the first state (the maximum irradiation area) and the second state (the irradiation area of one half the irradiation area in the first state). In the embodiment described above, the glasses reflection in the second image is reduced to the size of the corneal reflection that is originally detected in the first image. However, the purpose of the second variation is to reduce the glasses reflection in the second image to less than the glasses reflection in the first image.

Third variation

In the second variation, the irradiation area in the second state is set to a specified value regardless of the distance between the subject and the detection unit 7. However, the decision unit 46 may decide the irradiation area in the second state in accordance with the distance between the subject and the detection unit 7. That is to say, in the third variation, the irradiation area is decided using the distance in the same manner as the embodiment. However, unlike the embodiment, the size of the glasses reflection in the first image is not used.

Specifically, the storage unit 5 stores in advance an association relationship between the distance between the subject and the detection unit 7, and the irradiation area to be set. The decision unit 46 then refers to the association relationship, and decides the irradiation area in the second state in accordance with the distance between the subject and the detection unit 7. That is to say, in the line of sight detection system 100 according to this variation, the irradiation area irradiated from the light source 6 is switched between the first state and the second state decided from the association relationship set in advance for each frame.

In the embodiment described above, the irradiation area is decided in order to reduce the glasses reflection in the second image to the size of the corneal reflection that is to be originally detected in the first image using the size of the glasses reflection in the first image. However, the purpose of the third variation is to reduce the glasses reflection in the second image to less than the glasses reflection in the first image.

REFERENCE SIGNS LIST

| 100 | line of sight detection system |
| 1 | line of sight detection device |
| 2 | acquisition unit |
| 3 | control unit |
| 4 | identification unit |
| 5 | storage unit |
| 6, 106 | light source |
| 7 | detection unit |
| 101 | processor |
| 102 | ROM |
| 103 | RAM |
| 104 | HDD |
| 105 | communication device |
| 107 | detection device |
| 108 | input device |
| 109 | display device |
| 110 | medium reading device |
| 111 | bus |

**Claims**

1. A line of sight detection system comprising:

a light source that emits light toward a subject;
a light detector configured to detect reflected light from the subject;
a control unit configured to change an emission pattern of the light emitted from the light source toward the subject;
an identification unit configured to identify a line of sight position of the subject based on a change of the reflected light from the subject when the emission pattern of the light is changed; and
an output unit configured to output information of the line of sight position.

2. The line of sight detection system according to claim 1, wherein the light detector is an image capturing device configured to capture images of the subject.

3. The line of sight detection system according to claim 2, wherein the identification unit is configured to:

   identify the line of sight position using a first image captured when the emission pattern is in a first state and a second image captured when the emission pattern is in a second state.

4. The line of sight detection system according to claim 3, wherein
   the identification unit is configured to, when it is determined that an eyeglass reflection of the light occurs on a surface of eyeglasses worn by the subject in the first image, identify the emission pattern in the second state, and the control unit is configured to control the emission pattern of the light source to the second state based on the identified radiation pattern.

5. The line of sight detection system according to claim 4, wherein the identification unit is configured to:

   obtain a size of the eyeglass reflection in the first image, and identify the emission pattern of the second state in accordance with a distance between the subject and the light detector, and a size of the eyeglass reflection.

6. The line of sight detection system according to claim 5, wherein the identification unit is configured to:

   estimate a position of another eyeglass reflection that is present in the second image from a position of the eyeglass reflection in the first image,
   estimate a motion vector between a feature point in the first image and a corresponding feature point in the second image, and
   detect a corneal reflection on a cornea of the subject from a region separated from the position of the another eyeglass reflection and from a surrounding area in the second image.

7. The line of sight detection system according to any one of claims 1 to 6, wherein
   the light source includes an array of a plurality of light emitting elements, and
   the control unit is configured to control illumination of one or more light emitting elements of the plurality of light emitting elements so as to control the irradiation area.

8. The line of sight detection system according to claim 3, wherein the control unit is configured to:

   switch between the first state and the second state in accordance with a detection period of the light detector.

9. The line of sight detection system according to any one of claims 1 to 8, wherein light emitted from the light source is visible light.

10. The line of sight detection system according to any one of claims 1 to 8, wherein the light emitted from the light source includes light that is outside of a visible spectrum.

11. The line of sight detection system according to any one of claims 1 to 10, wherein emission pattern corresponds with an emission area of the light source.

12. The line of sight detection system according to any one of claims 1 to 10, wherein the emission pattern includes an irradiation pattern.

13. A line of sight detection method executed by a computer, the line of sight detection method comprising:

   changing an emission pattern of the light emitted from a light source toward a subject, the light source emitting light toward the subject;
   identifying a line of sight position of the subject based on a change of reflected light from the subject when the emission pattern of the light is changed, the reflected light being detected by a light detector; and
   outputting information of the line of sight position.

14. A line of sight detection program which, when executed on a computer causes the computer to carry out the line of sight detection method according to claim 13.

**15.** A storage medium storing the line of sight detection program of claim 14.

# FIG. 1

# FIG. 2A

# FIG. 2B

# FIG. 3

4

41

RECOGNITION UNIT

42

GLASSES REFLECTION
DETECTION UNIT

43

EXTRACTION UNIT

44

CORNEAL REFLECTION
DETECTION UNIT

45

PUPIL DETECTION UNIT

46

DECISION UNIT

47

LINE OF SIGHT
DETECTION UNIT

# FIG. 4

| DISTANCE (cm) | SIZE OF CORNEAL REFLECTION (PIXEL) |
|---|---|
| 40 | 8 |
| 50 | 5 |
| . . . | . . . |

# FIG. 5

```
                    ┌─────────────┐
                    │    START    │
                    └─────────────┘
                           │
                           ▼
        ┌──────────────────────────────────────┐
        │ CONTROL TURNING ON OF LIGHT SOURCE    │──  Op.1
        └──────────────────────────────────────┘
                           │
                           ▼
        ┌──────────────────────────────────────┐
        │            OBTAIN IMAGE               │──  Op.2
        └──────────────────────────────────────┘
                           │
                           ▼
        ┌──────────────────────────────────────┐
        │           OBTAIN DISTANCE             │──  Op.3
        └──────────────────────────────────────┘
                           │
                           ▼                Op.4
                    ◇─────────────────◇
                    │ IS DISTANCE WITHIN │      NO
                    │ PREDETERMINED RANGE? │────────►
                    ◇─────────────────◇
                           │ YES
                           ▼
        ┌──────────────────────────────────────┐
        │           DETECT EYE REGION           │──  Op.5
        └──────────────────────────────────────┘
                           │
                           ▼                Op.6
                    ◇─────────────────◇
                    │ NO GALASSES REFLECTION? │   NO
                    ◇─────────────────◇──────────►
                           │ YES
                           ▼
        ┌──────────────────────────────────────┐
        │        DETECT CORNEAL REFLECTION      │──  Op.7
        └──────────────────────────────────────┘
                           │
                           ▼
        ┌──────────────────────────────────────┐
        │ STORE DISTANCE AND SIZE OF CORNEAL    │──  Op.8
        │              REFLECTION               │
        └──────────────────────────────────────┘
                           │
                           ▼                Op.9
          NO        ◇─────────────────◇
        ◄───────────│   IS LEARNING    │
                    │ PROCESSING TERMINATED? │
                    ◇─────────────────◇
                           │ YES
                           ▼
                    ┌─────────────┐
                    │     END     │
                    └─────────────┘
```

# FIG. 6

START

CONTROL SO THAT IRRADIATION AREA BECOMES FISRT STATE — Op.11

OBTAIN FIRST IMAGE — Op.12

RECOGNIZE EYE REGION — Op.13

Op.14
HAS CORNEAL REFLECTION BEEN DETECTED?

— YES → DETECT PUPIL — Op.21

DETECT LINE OF SIGHT — Op.22

— NO

Op.15
HAS GLASSES REFLECTION BEEN DETECTED? — NO → (B)

YES — Op.16

STORE POSITION OF GLASSES REFLECTION

EXTRACT AND STORE FEATURE POINT — Op.17

OBTAIN DISTANCE — Op.18

DECIDE IRRADIATION AREA — Op.19

CONTROL SO THAT IRRADIATION AREA BECOMES SECOND STATE — Op.20

(A)

Op.23
IS PROCESSING TERMINATED? — NO

YES

END

# FIG. 7

(A)

↓

| OBTAIN SECOND IMAGE | ~ Op.31 |

↓

| RECOGNIZE EYE REGION | ~ Op.32 |

↓

| EXTRACT FEATURE POINT | ~ Op.33 |

↓

| CALCULATE MOTION VECTOR | ~ Op.34 |

↓

| ESTIMATE POSITION OF GLASSES REFLECTION REGION | ~ Op.35 |

↓

Op.36

HAS CORNEAL REFLECTION BEEN DETECTED? —— NO

↓ YES

| DETECT PUPIL | ~ Op.37 |

↓

| DETECT LINE OF SIGHT | ~ Op.38 |

↓

(B)

# FIG. 8A

# FIG. 8B

# FIG. 9

100

111

101
PROCESSOR

102
ROM

103
RAM

104
HDD

105
COMMUNICATION DEVICE

106
LIGHT SOURCE

107
DETECTION DEVICE

108
INPUT DEVICE

109
DEISPLAY DEVICE

110
MEDIUM READING DEVICE

Europäisches
Patentamt

European
Patent Office

Office européen
des brevets

**EUROPEAN SEARCH REPORT**

Application Number

EP 16 19 4340

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/086617 A1 (SENSOMOTORIC INSTR GES FÜR INNOVATIVE SENSORIK MBH [DE]) 18 June 2015 (2015-06-18) * abstract; claim 29; figures 1-3 * * page 18, line 20 - page 21, line 22 * ----- | 1-15 | INV. A61B3/113 ADD. A61B5/18 |
| X | SU GWON ET AL: "Gaze Tracking System for User Wearing Glasses", SENSORS, vol. 14, no. 2, 27 January 2014 (2014-01-27), pages 2110-2134, XP055238488, DOI: 10.3390/s140202110 * abstract; figure 1 * * Section: 2.Proposed method; figures 2-10 * ----- | 1,13-15 | |
| X,D | JP 2003 339642 A (CANON KK) 2 December 2003 (2003-12-02) * abstract * * paragraphs [0009] - [0012], [0014] - [0015] * ----- | 1,13-15 | |

TECHNICAL FIELDS SEARCHED (IPC)

A61B

The present search report has been drawn up for all claims

| Place of search | Date of completion of the search | Examiner |
|---|---|---|
| Munich | 17 March 2017 | Medeiros Gaspar, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 16 19 4340

This annex lists the patent family members relating to the patent documents cited in the above-mentioned European search report.
The members are as contained in the European Patent Office EDP file on
The European Patent Office is in no way liable for these particulars which are merely given for the purpose of information.

17-03-2017

| Patent document cited in search report | Publication date | Patent family member(s) | Publication date |
|---|---|---|---|
| WO 2015086617 A1 | 18-06-2015 | CN 105828700 A<br>EP 3079560 A1<br>JP 2016539717 A<br>US 2016270655 A1<br>WO 2015086617 A1 | 03-08-2016<br>19-10-2016<br>22-12-2016<br>22-09-2016<br>18-06-2015 |
| JP 2003339642 A | 02-12-2003 | NONE | |

EPO FORM P0459

For more details about this annex : see Official Journal of the European Patent Office, No. 12/82

**EP 3 158 921 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006167256 A

- JP 2003339642 A

**Non-patent literature cited in the description**

- **TAKEHIKO OHNO et al.** An Eye Tracking System Based on Eye Ball Model-Toward Realization of Gaze Controlled Input Device. *Information Processing Society of Japan Technical Reports 2001-HI-93,* 2001, 47-54